# EUROPEAN PATENT APPLICATION

(11) **EP 2 138 479 A1**
(43) Date of publication of application: **30.12.2009**
(21) Application number: 08305321.5
(22) Date of filing: 25.06.2008
(51) Int. Cl.: C07C 69/712, C11B 9/00

(54) **Phenyl glycolate derivatives**

(71) Applicant: V. Mane Fils S.A., 06620 Le Bar-sur-Loup (FR)
(72) Inventor: Plessis, Caroline, 06740, CHATEAUNEUF (FR); Mane, Jean, 06130, GRASSE (FR); Chanot, Jean-Jacques, 06530, SPERACEDES (FR)
(74) Representative: Cabinet Plasseraud

(57) **Abstract**

The invention relates to phenyl glycolate derivatives of formula (I) 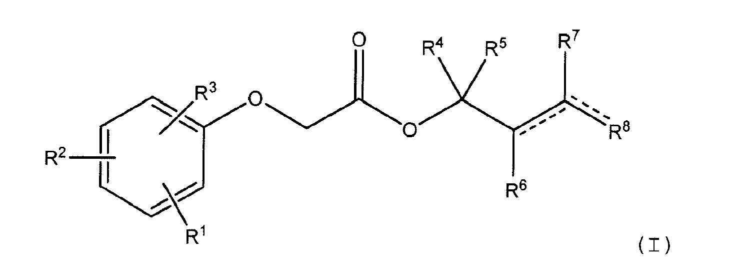 and their use in the field of perfumery.

## Description

### [FIELD OF THE INVENTION]

The present invention relates to the field of fragrances and flavours. More particularly, the invention relates to new phenyl glycolate derivatives and their use in the fields of perfumery and flavouring.

### [BACKGROUND]

Unsaturated glycolates belong to an important class of fragrant ingredients and much work has already been done to prepare known compounds, such as Cyclogalbanate® and allyl phenoxy acetate and similar derivatives. These compounds have a characteristic green (galbanum) and/or fruity (pineapple) note.

Developing new fragrant unsaturated glycolate derivatives, in particular phenoxy glycolates, is a huge challenge, since such compounds are quite stable to coloration, in particular compared to corresponding phenol compounds.

### [PROBLEM TO BE SOLVED]

The Applicant thus focused on the preparation of new unsaturated glycolate derivatives.

The need for new compounds is of great importance for the development of the fragrance industry, which recently had to face stricter international regulatory requirements about the use of certain materials, as well as environmental concerns and customer demands for improved performance. Developing new fragrant and/or flavouring compounds is also important for providing alternatives to already existing fragrant and/or flavouring compounds so as to minimize the risk of allergies due to repeated exposure to the same compounds. Providing new fragrant and/or flavouring compounds as well as means of manufacturing such compounds, is therefore an object of the invention.

In other words, it is an aim of the present invention to provide new fragrant compounds, in particular unsaturated glycolates.

### [SUMMARY OF THE INVENTION]

The invention is directed to novel compounds of formula (I) 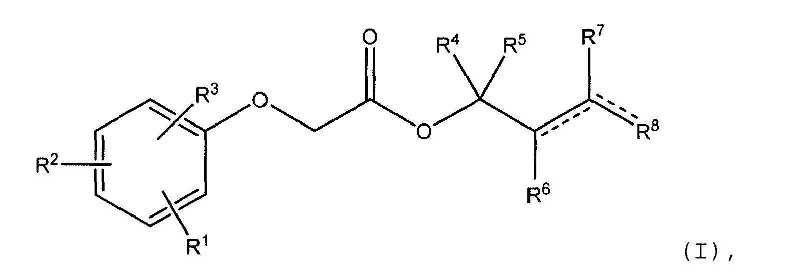 wherein
R¹ is H, alkyl, alkenyl, or alkoxy;
R² and R³ are independently alkyl, alkenyl, or alkoxy;
R⁴, R⁵, R⁶, and R⁷ are independently H, C₁-C₅ alkyl, or C₂-C₅ alkenyl; 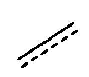 is a single or double bond, provided that one of the 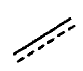 is a single bond and the other one is a double bond;
if R⁸ is attached to the double bond, it is H, C₁-C₅ alkyl, or C₂-C₅ alkenyl; and
if R⁸ is part of the double bond, it is -CR'R", wherein R' and R'' are independently H, C₁-C₄ alkyl, or C₂-C₄ alkenyl, with the proviso that R' and R'' together form at most a C₄-alkyl or C₄-alkenyl group.

The compounds of the invention exhibit interesting olfactive properties, such as sweet and/or spicy notes with sometimes animalic aspects. The compounds of the invention are therefore of particular interest in the field of perfumery.

### [DETAILED DESCRIPTION OF THE INVENTION]

As noted above, the invention relates to compounds of formula (I) 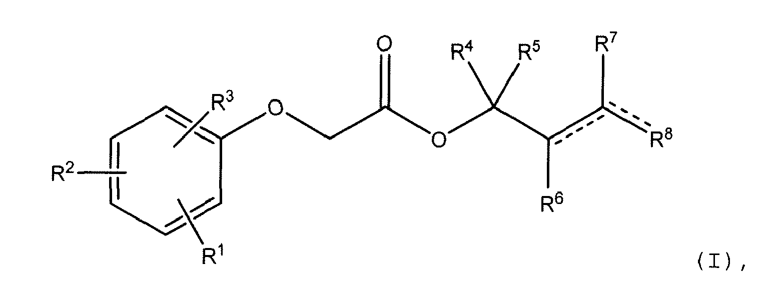 wherein
R¹ is H, alkyl, alkenyl, or alkoxy;
R² and R³ are independently alkyl, alkenyl, or alkoxy;
R⁴, R⁵, R⁶, and R⁷ are independently H, C₁-C₅ alkyl, or C₂-C₅ alkenyl;
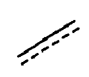 is a single or double bond, provided that one of
the 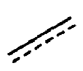 is a single bond and the other one is a double
bond;
if R⁸ is attached to the double bond, it is H, C₁-C₅ alkyl, or C₂-C₅ alkenyl; and
if R⁸ is part of the double bond, it is -CR'R", wherein R' and R'' are independently H, C₁-C₄ alkyl, or C₂-C₄ alkenyl, with the proviso that R' and R'' together form at most a C₄-alkyl or C₄-alkenyl group.

The invention includes all isomers of the compounds of formula (I).

R¹ is advantageously selected from H, C₁-C₅ alkyl, or C₂-C₅ alkenyl, or C₁-C₅ alkoxy.

Advantageously, R² and R³ are independently selected from C₁-C₅ alkyl, or C₂-C₅ alkenyl, or C₁-C₅ alkoxy.

C₁-C₅ alkyl comprises methyl, ethyl, propyl, butyl, and pentyl.

C₂-C₅ alkenyl comprises ethenyl, prop-1-enyl, allyl, butenyl, and pentenyl.

C₁-C₅ alkoxy comprises methoxy, ethoxy, propoxy, butoxy, and pentoxy.

According to an advantageous embodiment, R¹ is H and R² and R³ are C₁-C₅ alkyl, preferably C₁-C₄ alkyl, and even more preferably C₁-C₃ alkyl. Particularly interesting results are obtained when R² and R³ are both methyl or when R² is isopropyl and R³ is methyl.

According to another advantageous embodiment, R¹ is H, R² is C₁-C₅ alkyl, preferably C₁-C₄ alkyl, and even more preferably C₁-C₃ alkyl, and R³ is C₁-C₅ alkoxy, preferably C₁-C₃, and even more preferably methoxy. Particularly interesting results are obtained when R² is methyl and R³ is methoxy.

According to still another advantageous embodiment, R¹ is H, R² is C₂-C₅ alkenyl, preferably C₂-C₄ alkenyl, even more preferably C₂-C₃ alkenyl, and most preferably C₃ alkenyl, and R³ is C₁-C₅ alkoxy, preferably C₁-C₃, and even more preferably methoxy. Particularly interesting results are obtained when R² is methoxy and R³ is allyl or when R² is methoxy and R³ is prop-1-en-1-yl.

Further advantageous compounds are those wherein R¹ is H, and R² and R³ are independently C₂-C₅ alkenyl, preferably C₂-C₄ alkenyl, even more preferably C₂-C₃ alkenyl, and most preferably C₃ alkenyl. Very interesting results are obtained when R³ is allyl.

Another family of interesting compounds is obtained when R¹ is H, and R² and R³ are independently C₁-C₅ alkyl, preferably C₁-C₄ alkyl, more preferably C₁-C₃ alkyl, and most preferably methyl.

In one variant of the invention, the compound of formula (I) has formula (Ia) 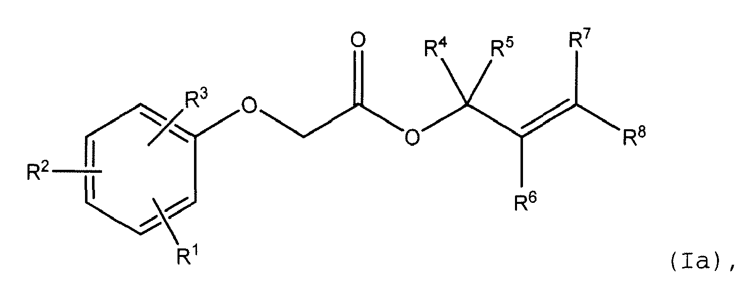 wherein R¹ to R⁷ are defined as in respect of formula (I), and R⁸ is H, C₁-C₅ alkyl, or C₂-C₅ alkenyl. The invention includes all isomers of the compounds of formula (Ia), in particular the E/Z isomers in respect of the double bond between CR⁶ and CR⁷.

According to another variant of the invention the compound of formula (I) has formula (Ib) 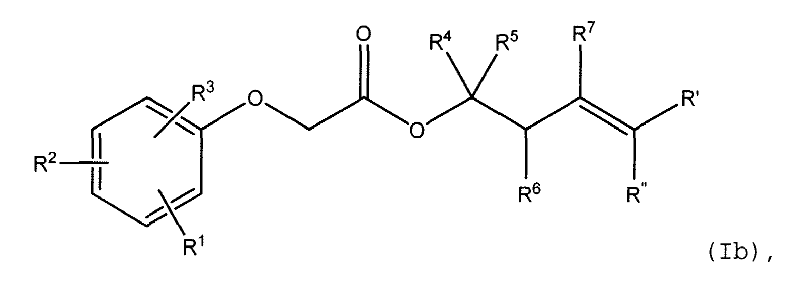 wherein R¹ to R⁷ are defined as in respect of formula (I), and R' and R'' are independently H, C₁-C₄ alkyl, or C₂-C₄ alkenyl, with the proviso that R' and R'' together form at most a C₄-alkyl or C₄-alkenyl group. The invention includes all isomers of the compounds of formula (Ia), in particular the E/Z isomers in respect of the double bond between CR⁷ and CR'R''.

Particularly interesting compounds of formula (Ib) are those wherein R' is H and R'' is ethyl.

The compounds of the invention exhibit interesting olfactive properties. Very unexpectedly and surprisingly, they do not show the typical green note that the skilled person would have expected, but rather sweet and/or spicy notes with sometimes animalic aspects.

A further object of the invention is thus the use of a compound of formula (I) as defined above, for the preparation of perfumed bases and concentrates, fragrances, perfumes; topic compositions; cosmetic compositions, such as face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; and cleaning products, such as softeners, detergents, air deodorizers and household cleaning supplies.

The invention is also directed to the use of a compound of formula (I) as defined above, as flavouring agent for the preparation of foodstuffs, drinks, and tobacco. The foodstuffs and drinks are preferably selected from the group consisting of dairy products, ice creams, soups, sauces, dips, dishes, meat products, culinary assistances, salted biscuits, snacks, soft drinks, beers, wines and spirits.

The invention is also directed to the use of a compound of formula (I) as defined above, as masking agent of odours and/or flavours, e.g. in pharmaceutical, cosmetic or food compositions.

The invention also provides the use of a compound of formula (I) as defined above, in combination with other perfuming or flavouring ingredients, solvents or additives or fixatives.

The compounds of the invention may be used in a concentration comprised in a range from 0.001% to 99% in weight, preferably from 0.1% to 50% in weight, more preferably from 0.1% to 30% in weight. It is known by the man skilled in the art that these values depend on the nature of the composition/article to be perfumed and/or flavoured, the desired intensity of the perfume and/or flavour, and of the nature of the other ingredients present in said composition or article. According to a preferred embodiment of the invention, compounds are used in an olfactory effective amount.

### [DEFINITIONS]

The terms "fragrance" and "fragrant", as used herein, are used interchangeably whenever a compound or a mixture of compounds is referred to, which is intended to pleasantly stimulate the sense of smell.

The terms "flavour" and "flavouring", as used herein, are used interchangeably whenever a compound or a mixture of compounds is referred to, which is intended to stimulate the sense of taste and smell. Also in the meaning of the invention, the term "flavouring" relates to the flavouring of any liquid or solid, human or animal, in particular of drinks, dairy products, ice creams, soups, sauces, dips, dishes, meat products, culinary assistances, salted biscuits or snacks. It also means the flavouring of beers, wines and tobaccos.

The term "olfactory effective amount", as used herein, means a level or amount of fragrant/flavouring compound present in a material at which the incorporated compound exhibits a sensory effect.

By the term "masking" is meant reducing or eliminating malodour or bad flavour perception generated by one or more molecules entering in the composition of a product.

The term "isomer", in the present invention, means molecules having the same chemical formula, which means same number and types of atoms, but in which the atoms are arranged differently. The term "isomer" includes structural isomers, geometric isomers, optical isomers and stereoisomers. It particularly includes the *Z*/*E* isomers of the compounds of formulae (I), (Ia), and (Ib).

The term "alkyl" or "alkyl group", in the present invention, means any linear or branched saturated hydrocarbon chain, having preferably 1, 2, 3, 4 or 5 carbon atoms and herein referred to as C₁₋₅ alkyl group, such as for example methyl, ethyl, *n*-propyl, *iso*-propyl, *n*-butyl, *sec*-butyl, *iso*butyl, *tert*-butyl, and pentyl.

The term "alkenyl" or "alkenyl group", in the present invention, means any linear or branched mono or poly unsaturated hydrocarbon chain, having preferably 2, 3, 4 or 5 carbon atoms and herein referred to as C₂₋₅ alkenyl group, such as for example ethenyl, prop-1-enyl, allyl, but-1-enyl, but-2-enyl, or pentenyl.

The term "alkoxy" or "alkoxy group", in the present invention, means a oxygen radical substituted with any linear or branched saturated hydrocarbon chain, having preferably 1, 2, 3, 4 or 5 carbon atoms and herein referred to as C₁₋₅ alkoxy group, such as for example methoxy, ethoxy, *n*-propoxy, *iso*-propoxy, *n*-butoxy, *sec*-butoxy, *iso*-butoxy, *tert*-butoxy, and pentoxy.

The present invention will be better understood with reference to the following examples. These examples are intended to representative of specific embodiments of the invention, and are not intended as limiting the scope of the invention.

### Example 1: Preparation of (3,5-dimethyl-phenoxy)-acetic acid allyl ester

A solution of 3,5-dimethyl-phenol in refluxing acetone is treated overnight with chloroacetic acid allyl ester (1.1 eq.) and potassium carbonate (1 eq.).
The mixture is then poured into a 50:50 water/t-butyl methyl ether mixture and the aqueous phase is extracted twice with *t*-butyl methyl ether. The combined organic phases are washed with brine and dried over magnesium sulphate. The solvents are evaporated and the crude product is purified by distillation to give (3,5-dimethyl-phenoxy)-acetic acid allyl ester.
Odour description: musky, hot iron, not powerful.
¹H-NMR (200 MHz, CDCl₃) : δ (ppm) 2.26 (s, 3H), 2.29 (s, 3H), 4.63 (s, 2H), 4.71 (td, *J* = 1.2 Hz, *J* = 5.8 Hz, 2H), 5.22-5.42 (m, 2H), 5.94 (tdd, *J* = 5.8 Hz, *J* = 10.3 Hz, *J* = 17.0 Hz, 1H), 6.55 (br s, 2H), 6.65 br s, 1H). ¹³C-NMR (50 MHz, CDCl₃): δ (ppm) 21.35, 65.20, 65.67, 112.34, 118.89, 123.48, 131.45, 139.26, 157.75, 168.75. IR (film, cm⁻¹): 687m, 830m, 929m ; 987m, 1032w, 1096m, 1157s, 1171s, 1200s, 1277m, 1299m, 1324s, 1383w, 1442m, 1473m, 1596s, 1615m, 1739m, 1764s, 2922m, 3020w. MS [e/m (%)]: 220 (M+, 99), 135 (69), 105 (100), 103 (17), 91 (16), 79 (29), 77 (36), 41 (25), 39 (19).

### Example 2: Preparation of (2-isopropyl-5-methyl-phenoxy)-acetic acid allyl ester

It is prepared from thymol according to example 1.
Odour description: Costus.
¹H-NMR (200 MHz, CDCl₃): δ (ppm) 1.25 (d, *J* = 6.9 Hz, 6H), 2.32 (s, 3H), 3.40 (sept., *J* = 6.9 Hz, 1H), 4.68 (s, 2H), 4.73 (td, *J* = 1.3 Hz, *J* = 5.8 Hz, 2H), 5.22-5.42 (m, 2H), 5.95 (tdd, *J* = 5.7 Hz, *J* = 10.4 Hz, *J* = 17.1 Hz, 1H), 6.57 (br s, 1H), 6.81 (br d, *J* = 7.7 Hz, 1H), 7.14 (br d, *J* = 7.7
Hz, 1H).
¹³C-NMR (50 MHz, CDCl₃): δ (ppm) 21.72, 23.22, 27.03, 66.07, 112.77, 119.21, 122.75, 126.71, 131.99, 135.02, 136.71, 155.39, 169.31. IR (film, cm⁻¹): 813m, 932m, 986m, 1071m, 1102m, 1120m, 1167s, 1194s, 1279m, 1416m, 1445m, 1507m, 1614w, 1740m, 1765s, 2871w, 2962m. MS [e/m (%)]: 248 (M+, 100), 233 (68), 207 (18), 187 (12), 179 (23), 149 (59), 147 (22), 121 (53), 105 (50), 91 (42), 77 (21), 41 (62), 39 (30).

### Example 3: Preparation of (4-Allyl-2-methoxy-phenoxy)-acetic acid allyl ester

It is prepared from eugenol according to example 1.
Odour description: Eugenol, carnation-type, woody.
Not as powerful as Eugenol, iso-Eugenol nor Methyl-Diantilis^{®}.
¹H-NMR (200 MHz, CDCl₃): δ (ppm) 3.32 (br d, *J* = 6.7 Hz, 2H); 3.85 (s, 3H); 4.64-4.70 (m, 2H); 4.68 (s, 2H); 5.0-5.05 (m, 1H); 5.05-5.13 (m, 1H); 5.19-5.37 (m, 2H); 5.80-6.03 (m, 2H); 6.64-6.80 (m, 3H).
¹³C-NMR (50 MHz, CDCl₃): δ (ppm) 39.69; 55.72; 65.55; 66.60; 112.47; 114.63; 115.65; 118.76; 120.28; 131.43; 134.43; 137.30; 145.46; 149.47; 168.72. IR (film, cm⁻¹): 749w, 805m, 851w, 919m, 991m, 1036s, 1072m, 1148s, 1189s, 1262s, 1420m, 1464m, 1512s, 1593m, 1639w, 1738s, 1762s, 2836w, 2939m, 2977w, 3003w, 3079w. MS [e/m (%)]: 262 (M+, 100), 163 (25), 115 (14), 109 (9), 103 (17), 91 (17), 77 (10), 41 (22).

### Example 4: (2-Methoxy-4-propenyl-phenoxy)-acetic acid allyl ester

It is prepared from *iso*-eugenol according to example 1.
Odour description: Spicy (*iso*-Eugenol), leathery, carnation, saffron, fruity (plums, dried plums), woody (Vetyver), sweet. Not as powerful as Eugenol, iso-Eugenol nor Methyl-Diantilis^{®}.
It consists in a (30:70) *Z*/*E* mixture of isomers. IR (film, cm⁻¹): 784w, 816w, 859w, 933w, 966m, 985m, 1036m, 1071m, 1144s, 1191s, 1259s, 1272s, 1259s, 1272s, 1298m, 1416m, 1463m, 1512s, 1585w, 1602w, 1738m, 1762s, 2916w, 2938w, 3019w.

### Z-Isomer:

¹H-NMR (200 MHz, CDCl₃): δ (ppm) 1.89 (dd, *J* = 1.6 Hz, *J* = 7.3 Hz, 3H), 3.87 (s, 3H), 4.64-4.73 (m, 2H), 4.69 (s, 2H), 5.18-5.38 (m, 2H), 5.72 (ddd, *J* = 8.1 Hz, *J* = 12.5 Hz, *J* = 14.4 Hz, 1H), 6.07 (q, J = 6.4 Hz, 1H), 6.34-6.40 (m, 1H), 6.72 (br s, 1H), 6.77-6.82 (m, 1H), 6.82-6.86 (m, 1H). ¹³C-NMR (50 MHz, CDCl₃): δ (ppm) 14.55, 55.80, 65.64, 66.49, 112.84, 114.10, 118.36, 121.15, 125.87, 129.27, 131.44, 132.33, 145.77, 149.15, 168.65. MS [e/m (%)]: 262 (M+, 100), 163 (47), 162 (16), 115 (16), 107 (29), 103 (13), 91 (21), 77 (10), 41 (19), 39 (10).

### E-Isomer:

¹H-NMR (200 MHz, CDCl₃): δ (ppm) 1.85 (dd, *J* = 1.4 Hz, *J* = 6.4 Hz, 3H), 3.87 (s, 3H), 4.64-4.73 (m, 2H), 4.69 (s, 2H), 5.18-5.38 (m, 2H), 5.91 (tdd, *J* = 5.8 Hz, *J* = 10.4 Hz, *J* = 17.3 Hz, 1H), 6.14 (q, *J* = 6.3 Hz, 1H), 6.26-6.31 (m, 1H), 6.77 (br s, 1H), 6.77-6.82 (m, 1H), 6.88-6.91 (m, 1H). ¹³C-NMR (50 MHz, CDCl₃): δ (ppm) 18.29, 55.75, 65.64, 66.55, 109.26, 114.49, 118.36, 118.85, 124.46, 130.36, 131.44, 132.80, 146.22, 149.58, 168.65. MS [e/m (%)]: 262 (M+, 100), 163 (43), 162 (16), 115 (13), 107 (24), 103 (11), 91 (17), 77 (8), 41 (16), 39 (9).

### Example 5: (2-Allyl-phenoxy)-acetic acid allyl ester

It is prepared from 2-allyl-phenol according to example 1. Odour description: Vanilla, plastic.
¹H-NMR (200 MHz, CDCl₃): δ (ppm) 3.48 (br d, *J* = 6.6 Hz, 2H); 4.68 (s, 2H); 4.71 (dt, *J* = 1.2 Hz, *J* = 5.9Hz, 2H); 5.02-5.07 (m, 1H); 5.07-5.15 (m, 1H); 5.23-5.40 (m, 2H); 6.04 (ddt, *J* = 5.8 Hz, *J* = 9.2 Hz, *J* = 15.1Hz, 1H); 5.94 (tdd, *J* = 5.7 Hz, *J* = 10.4 Hz, *J* = 17.1 Hz, 1H); 6.7-6.8 (m, 1H); 6.91-7.01 (m, 1H); 7.12-7.23 (m, 2H).
¹³C-NMR (50 MHz, CDCl₃): δ (ppm) 34.23; 65.59; 65.67; 111.39; 115.50; 118.88; 121.66; 127.21; 129.26; 130.14; 131.42; 136.76; 155.51; 168.66. IR (film, cm⁻¹): 754s, 919m, 989m, 1072m, 1092m, 1129m, 1194s, 1276m, 1296m, 1441m, 1455m, 1493s, 1589m, 1601w, 1639w, 1739s, 1764s, 2980w, 3079w.
MS [e/m (%)]: 232 (M+, 95), 145 (100), 133 (22), 131 (27), 117 (20), 115 (75), 105 (17), 91 (62), 77 (20), 41 (53), 39 (37).

### Example 6: (2-Methoxy-4-methyl-phenoxy)-acetic acid allyl ester

It is prepared from 2-methoxy-4-methyl-phenol according to example 1.
Odour description: Vanilla, spicy (*iso*-Eugenol).
¹H-NMR (200 MHz, CDCl₃): δ (ppm) 2.29 (s, 3H), 3.85 (s, 3H), 4.65-4.71 (m, 2H), 4.66 (s, 2H), 5.20-5.38 (m, 2H), 5.91 (tdd, *J* = 5.8 Hz, *J* = 10.3 Hz, *J* = 17.1 Hz, 1H), 6.62-6.79 (m, 3H).
¹³C-NMR (50 MHz, CDCl₃) δ (ppm) 20.99, 55.73, 65.59, 66.76, 113.10, 114.81, 118.80, 120.73, 131.49, 132.34, 144.99, 149.38, 168.84. IR (film, cm⁻¹): 800w, 929w, 987w, 1037m, 1071w, 1148s, 1156s, 1191s, 1269s, 1465w, 1513s, 1593w, 1737m, 1762s, 2939w.
MS [e/m (%)]: 236 (M+, 100), 151 (11), 137 (46), 136 (39), 109 (20), 91 (40), 77 (16), 65 (15), 41 (21), 39 (17).

### Example 7: p-Tolyloxy-acetic acid hex-3-enyl ester

It is prepared from p-cresol and chloroacetic acid *cis*-3-hexenyl ester according to example 1.
Odour description: Animal, leathery.
¹H-NMR (200 MHz, CDCl₃) δ (ppm) 0.97 (t, *J* = 7.5 Hz, 3H), 2.05 (br quint, *J* = 7.6 Hz, 2H), 2.29 (s, 3H), 2.41 (br q, *J* = 7.0 Hz, 2H), 4.20 (t, *J* = 6.9 Hz, 2H), 4.59 (s, 2H), 5.21-5.37 (m, 1H), 5.44-5.60 (m, 1H), 6.76-6.85 (m, 2H), 7.04-7.13 (m, 2H).
¹³C-NMR (50 MHz, CDCl₃): δ (ppm) 14.15, 20.42, 20.55, 26.61, 64.62, 65.52, 114.50 (2C), 123.89, 129.92 (2C), 130.96, 134.84, 155.71, 169.09.
IR (film, cm⁻¹): 724m, 818m, 996m, 1086s, 1191s, 1290s, 1391w, 1443m, 1458m, 1512s, 1589w, 1391m, 1737s, 1762s, 2874m, 2932m, 2964s, 3012m.
MS [e/m (%)]: 248 (M+, 38), 166 (100), 121 (35), 108 (14), 107 (12), 91 (54), 82 (22), 77 (14), 67 (36), 65 (21), 55 (35), 41 (28).

### Example 8: (2-Methoxy-4-methyl-phenoxy)-acetic acid hex-3-enyl ester

It is prepared from 2-methoxy-4-methyl-phenol and chloroacetic acid *cis*-3-hexenyl ester according to example 1. Odour description: Vanilla, smokey.
¹H-NMR (200 MHz, CDCl₃) δ (ppm) 0.95 (t, *J* = 7.5 Hz, 3H), 2.03 (br quintuplet, *J* = 7.6 Hz, 2H), 2.29 (s, 3H), 2.39 (br q, *J* = 7.1 Hz, 2H), 3.85 (s, 3H) ppm 4.18 (t, *J* = 7.0 Hz, 2H), 4.64 (s, 2H) ppm 5.27 (m, 1H), 5.49 (m, 1H), 6.61-6.77 (m, 3H).
¹³C-NMR (50 MHz, CDCl₃): δ (ppm) 14.13, 20.52, 21.0, 26.6, 55.75, 64.50, 66.74, 113.09, 114.71, 120.73, 123.22, 132.26, 134.74, 145.07, 149.38, 169.17.
IR (film, cm⁻¹): 798w, 817w, 997w, 1038s, 1075m, 1148s, 1156s, 1191s, 1268s, 1414w, 1465m, 1513s, 1593w, 1735m, 1762s, 2874w, 2935m, 2963m, 3010w.
MS [e/m (%)]: 278 (M+, 100), 196 (55), 151 (11), 137 (44), 136 (27), 109 (15), 91 (26), 77 (12), 67 (17), 65 (11), 55 (28), 41 (22).

### Example 9: Fragrance composition containing the derivatives obtained in example 3 and in example 4

A lilac type accord was prepared from the following ingredients:

| **Ingredients** | **Accord A** | **Accord B** | **Accord C** | **Accord D** |
|---|---|---|---|---|
| BENZYL SALICYLATE | 60 | 60 | 60 | 60 |
| PHENYL ETHYL ALCOOL | 240 | 240 | 240 | 240 |
| VANILLINE | 30 | 30 | 30 | 30 |
| HELIOTROPINE | 70 | 70 | 70 | 70 |
| IONONE ALPHA | 20 | 20 | 20 | 20 |
| TERPINEOL BI RECT | 40 | 40 | 40 | 40 |
| BENZYL ACETATE | 40 | 40 | 40 | 40 |
| DPG | 500 | 460 | | 460 |
| (4-Allyl-2-methoxy-phenoxy)-acetic acid allyl ester **(Example 3)** | | **500** | | |
| (2-Methoxy-4-propenyl-phenoxy)-acetic acid allyl ester **(Example 4)** | | | **500** | |
| *Iso*-Eugenol | | | | 20 |

Despite the fact that (4-allyl-2-methoxy-phenoxy)-acetic acid allyl ester and (2-methoxy-4-propenyl-phenoxy)-acetic acid allyl ester are less powerful than Eugenol or *iso*-Eugenol, adding them to the accord A instead of iso-Eugenol leads to a more floral, carnation-lilac note. Moreover, (4-allyl-2-methoxy-phenoxy)-acetic acid allyl ester (Accord B) brings also a special vegetal fresh facets and is thus preferred for such an accord.
Iso-Eugenol gives a more phenolic vanilla-type note.

### Example 10: Fragrance composition containing the derivative obtained in example 4

A "Malaga Rhum-Raisin" accord was prepared from the following ingredients:

| **Ingredients** | **Accord A** | **Accord B** |
|---|---|---|
| NOIX DE MUSCADE ESSENCE | 15 | 15 |
| CINNAMIQUE ALDEHYDE | 4 | 4 |
| EUGENOL RECTIFIE VMF | 15 | 15 |
| ETHYL VANILLINE | 120 | 120 |
| ETHYL MALTOL | 30 | 30 |
| AMYL ACETATE VMF | 90 | 90 |
| ETHYL PROPIONATE | 210 | 210 |
| ETHYL PHTALATE | 516 | 16 |
| **(2-Methoxy-4-propenyl-phenoxy)-acetic acid allyl ester** (Example 4) | - | **500** |

Adding (2-methoxy-4-propenyl-phenoxy)-acetic acid allyl ester to accord A imparts a really more fruity (grapes), spicy, gourmand facets to the note.

These 2 compositions, containing (A) or not (B) the (2-methoxy-4-propenyl-phenoxy)-acetic acid allyl ester from example 4, were used to perfume a candle at usual dilution, known to the person of the art. Again, the impact of the addition of the (2-methoxy-4-propenyl-phenoxy)-acetic acid allyl ester in formula A is important. Adding (2-methoxy-4-propenyl-phenoxy)-acetic acid allyl ester increases considerably the diffusivity of the perfume and brings a culinary, spicy, "Christmas spirit" facets.

### Example 11: Fragrance composition containing the derivative obtained in example 4

A Basil accord was prepared from the following ingredients:

| **Ingredients** | **Accord A** | **Accord B** |
|---|---|---|
| LINALOL DE HO | 500 | 500 |
| EUCALYPTOL | 20 | 20 |
| HEXENOL CIS 3 | 20 | 20 |
| OCIMENE | 15 | 15 |
| TERPINEOL DROIT CRIST | 30 | 30 |
| LIMONENE | 100 | 100 |
| BORNYL ACETATE | 20 | 20 |
| CITRAL PUR DU | | |
| LITSEA | 6 | 6 |
| GERANYL ACETATE | 5 | 5 |
| ESTRAGOL 100% | 14 | 14 |
| | | |
| **(2-Methoxy-4-propenyl-phenoxy)-acetic acid allyl ester** (Example 4) | **270** | - |
| **DPG** | - | **270** |

Adding (2-methoxy-4-propenyl-phenoxy)-acetic acid allyl ester to the accord (Accord A) gives a more vegetal note, very natural, with earthy aspects. Thus accord A is more characteristic to a basil accord.

These 2 compositions, containing (A) or not (B) the (2-methoxy-4-propenyl-phenoxy)-acetic acid allyl ester from example 4, were used in a Chypre composition, according to the following ingredients:

| **Ingredients** | **Composition A** | **Composition B** |
|---|---|---|
| LAVANDE ESS URSS | 80 | 80 |
| LITSEA CUBEBA ESS | 20 | 20 |
| BERGAMOTE ESS | 150 | 150 |
| CITRON ESS | 200 | 200 |
| GERANIUM CHINE ESS | 5 | 5 |
| PATCHOULI ESS | 30 | 30 |
| VETYVER ESS HAITI | 10 | 10 |
| MOUSSE DE CHENE VMF ABS 50% EMKANOL | 20 | 20 |
| BENZYL ACETATE | 10 | 10 |
| LINALYL ACETATE GLIDDEN | 154 | 154 |
| CARVONE L VMF USA | 10 | 10 |
| COUMARINE | 10 | 10 |
| LINALOL HLR | 170 | 170 |
| INDOL | 1 | 1 |
| ALPHA METHYLIONONE | 30 | 30 |
| | | |
| **BASIL ACCORD A** | **40** | |
| **BASIL ACCORD B** | | **40** |

Adding accord A, containing (2-methoxy-4-propenyl-phenoxy)-acetic acid allyl ester, to the Chypre composition imparts very interesting, fresher, aromatic facets, with shiny, fusing, sparkling aspects.

## Claims

1. A compound of formula (I) 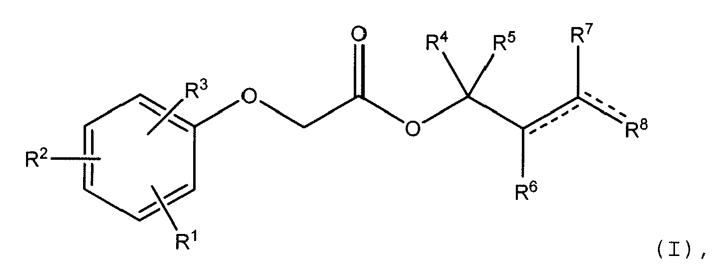 wherein
R¹ is H, alkyl, alkenyl, or alkoxy;
R² and R³ are independently alkyl, alkenyl, or alkoxy;
R⁴, R⁵, R⁶, and R⁷ are independently H, C₁-C₅ alkyl, or C₂-C₅ alkenyl;
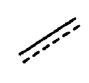 is a single or double bond, provided that one of
the 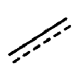 is a single bond and the other one is a double bond;
if R⁸ is attached to the double bond, it is H, C₁-C₅ alkyl, or C₂-C₅ alkenyl; and
if R⁸ is part of the double bond, it is -CR'R", wherein R' and R'' are independently H, C₁-C₄ alkyl, or C₂-C₄ alkenyl, with the proviso that R' and R'' together form at most a C₄-alkyl or C₄-alkenyl group

2. The compound of claim 1, wherein R¹ is H.

3. The compound of claim 1 or 2, wherein R⁴, R⁵, R⁶, and R⁷ are H.

4. The compound of claim 1 having formula (Ia) 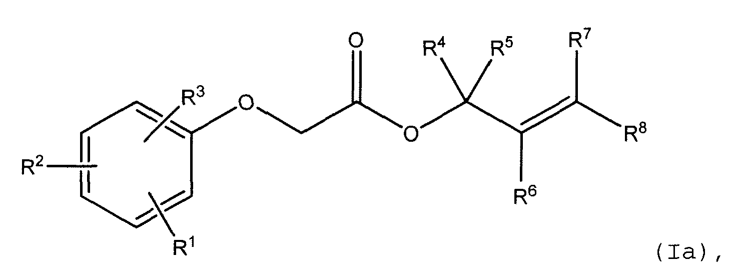 wherein R¹ to R⁸ are defined as in claim 1.

5. The compound of claim 4, wherein R⁷ and R⁸ are H.

6. The compound of claim 4, wherein R⁴, R⁵, R⁶ and R⁷ are H.

7. The compound of claim 4, wherein said compound is selected from the group consisting of (3,5-dimethyl-phenoxy)-acetic acid allyl ester, (2-isopropyl-5-methyl-phenoxy)-acetic acid allyl ester, (4-allyl-2-methoxy-phenoxy)-acetic acid allyl ester, (2-methoxy-4-propenyl-phenoxy)-acetic acid allyl ester, (2-allyl-phenoxy)-acetic acid allyl ester, and (2-methoxy-4-methyl-phenoxy)-acetic acid allyl ester.

8. The compound of claim 1 having formula (Ib) 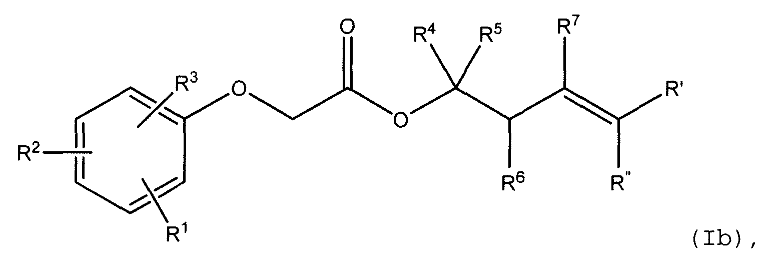 wherein R¹ to R⁷ and R' and R'' are defined as in claim 1.

9. The compound of claim 8, wherein R' is ethyl.

10. The compound of claim 8 or 9, wherein R⁴, R⁵, R⁶, and R⁷ are H.

11. The compound of claim 8, wherein said compound is selected from the group consisting of *p*-tolyloxy-acetic acid hex-3-enyl ester and (2-methoxy-4-methyl-phenoxy)-acetic acid hex-3-enyl ester.

12. Use of a compound according to any of claims 1 to 11, as a fragrant agent or as a flavouring agent.

13. Use of a compound according to any of claims 1 to 11, as a masking agent of odours and/or flavours.

14. Use according to claim 12 or 13 **characterized in that** the compound is comprised in a composition selected from pharmaceutical, cosmetic or food compositions.

15. Use according to any of claims 12 to 14
**characterized in that** the compound is used in combination with other perfuming or flavouring ingredients, solvents, additives or fixatives.
